# EUROPEAN PATENT APPLICATION

(11) **EP 4 480 513 A1**
(43) Date of publication of application: **25.12.2024**
(21) Application number: 23180855.1
(22) Date of filing: 22.06.2023
(51) Int. Cl.: A61M 1/06, A61B 5/00, G02B 27/28

(54) **PERSONAL CARE DEVICE**

(71) Applicant: Koninklijke Philips N.V., 5656 AG Eindhoven (NL)
(72) Inventor: PERRONE, Antonio Luigi, Eindhoven (NL); JOHNSON, Mark Thomas, Eindhoven (NL); GERHARDT, Lutz Christian, Eindhoven (NL)
(74) Representative: Philips Intellectual Property & Standards

(57) **Abstract**

The invention relates to a personal care device (100), a method for operating a personal care device (100) and a computer program product. The personal care device (100) comprises a movable component (110), an actuator (120) configured to move the movable component (110) and a polarization element (120). The movement of the movable component (110) changes a polarization parameter of the polarization element (130). The personal care device (100) may be a breast pump. A system (200) comprises the personal care device (100), a light source (210) and a light sensor (220).

## Description

### FIELD OF THE INVENTION

The present invention relates to the field of personal care devices.

### BACKGROUND OF THE INVENTION

Recently, personal care devices (e.g., toothbrushes, breast pumps, shavers, etc.) are being adapted to be used for a number of secondary purposes. That is, there is an increasing interest in personal care devices that have enhanced functionality beyond their primary purpose.

Specifically, many personal care devices are incorporating the use of a camera/optical system to image an area to which the device is applied. Imaging may be used to assess effectiveness of the device in real-time, analyse the use of the device over time, and for providing diagnostic information of the anatomy to which the personal care device is applied. For example, an imaging system may be used on a toothbrush to give real-time or post-hoc feedback to the user, or to identify cavities.

However, means of imaging used by such devices either do not provide images of an adequate resolution, or are bulky and costly. Furthermore, there is a desire in some cases for the tissue of the user to be imaged at different depths, to be able to identify (deeper tissue) pathologies and/or structures beneath the surface of the user.

Therefore, there exists a need for a personal care device that facilitates enhanced imaging of the user's subsurface tissue structures.

### SUMMARY OF THE INVENTION

The invention is defined by the claims.

According to examples in accordance with an aspect of the invention, there is provided a personal care device, comprising:
a movable component;
an actuator configured to move the movable component;
a polarization element configured to polarize light incident on the polarization element; and
wherein the movement of the movable component changes a polarization parameter of the polarization element.

Embodiments of the invention are based on the realisation that the movement of components of the personal care device itself may be used to change the type of polarization of a polarization provided thereon. That is, embodiments of the invention provide a personal care device having a polarization element that polarizes incident light differently as an actuator of the personal care device moves a movable component, which alters a polarization parameter (i.e. a degree, orientation, etc. of polarization), for example by rotating or altering dimensions of the polarization element.

By way of explanation, using polarized light as opposed to non-polarized light for imaging tissue may result in an increase in optical depth penetration. For example, depth penetration may increase from 1-2 mm as in typical optical methods, to 6-8mm when using polarized light. This is particularly beneficial for breast tissue, in which structures may develop deep within the tissue. By imaging the same tissue with light having different polarizations a horizontal resolution of a resultant image may be improved. Accordingly, the polarization filter provided by the present invention may be used to facilitate this functionality.

Put another way, a need for additional components (i.e. a dedicated actuator) for manipulation of a polarization filter may be avoided. Indeed, many personal care devices typically incorporate one or more moving parts and actuators in order to fulfil their primary function. For example, a breast pump may have a hydraulic or pneumatic pump to provide a controlled air flow suctioning force, and a toothbrush may have a motor to provide vibrations to a cleaning unit (e.g. brush head). Movement of these components (e.g. brush head, piston of a pump to pump air as medium, shaver cutter rotations) can be utilised to manipulate/control a polarization filter, without the need for additional/further costly and complex components.

In a particular embodiment, the polarization element may be flexible such that one or more dimensions of the polarization element may be changed, thereby changing a degree of polarization of light incident to the polarization element.

One way in which the invention may be realised is by providing a polarization element that is flexible, and that has polarization parameters based on its dimensions. That is, many typical polarization elements polarize light differently based on a length of an optical path of the polarization element. By altering the dimension of the polarization element, an optical path may be altered and thus a polarization parameter (e.g. a degree and/or type of polarization) may be changed.

More specifically, the actuator may be configured to move the movable component so as to compress the polarization element. Additionally or alternatively, the actuator may be configured to move the moveable component so as to stretch the polarization element.

In these ways, the dimensions of the polarization element may be changed, and thus a polarization parameter of the polarization element altered. It then follows that light incident to the polarization element when compressed/stretched by the moveable component is different to when it has not been compressed/stretched.

In some embodiments, the actuator may be configured to move the moveable component so as to rotate the polarization element.

Of course, another way in which movement of the moveable component may impact the polarization element is to rotate/turn the polarization element. For example, this may simply change the orientation of linear polarization. Alternatively, a rotation may be used to select a different aspect of the polarization element to which the light is incident, thus changing the type of polarization. That is, different faces of the polarization element may provide a clockwise circular polarization filter, an anticlockwise circular polarization filter, and/or a linear polarization filter.

In any case, the movable component may rotate such that light incident to the polarization element is different polarized. This may be performed alternatively or in addition to altering dimensions of the polarization element.

In further embodiments, the polarization element may comprise a cavity for containing a fluid, a length of an optical path of the polarization element depending on a volume of fluid within the polarization element. In this case, the actuator may be configured to move the movable component so as to change the volume of fluid in the cavity.

Put another way, yet another additional or alternative method by which the movement of the moveable component may change the polarization parameter of the polarization filter is by altering an optical path by changing a volume of fluid within (a cavity of) the polarization element. The fluid may be introduced to the cavity to change an effective optical path of the cavity, and therefore an optical path of the polarization element. This may be particularly advantageous for example, when the actuator is a pump (e.g. a pump of a breast pump or an oral flossing device).

In some embodiments, the actuator and/or the movable component may be further configured for operation of the personal care device.

Indeed, the actuator and/or moveable component may be the actual device that forms part of the operation of the personal care device (e.g., the rotation of a motor within an electric toothbrush, the piston of a pump of a breast pump, etc.). Thus, (periodic, cyclic or oscillating) movement of the typical personal care device itself can be used to alter the polarization parameter of the polarization element. This may be particularly advantageous where imaging (via the polarization element) is performed synchronously with movement of the personal care device. That is, when the polarization parameters are to be dynamically adjusted in a synchronous manner with the personal care device, it may be beneficial to leverage the movement of the actuator and/or movable component that is responsible for operation of the personal care device.

In some embodiments, the personal care device may further comprise a control unit configured to manipulate the polarization element to change the polarization parameter by controlling the actuator to move the movable component.

That is, a control unit may be provided to actively control the actuator in order to achieve the desired change/manipulation of the polarization element to give rise to the desired polarization parameter.

According to exemplary embodiments, the actuator and/or movable component may be a pump. More particularly, the personal care device may be a breast pump, and the actuator and/or movable component may be a pump configured to move the moveable component.

This invention may be particularly suitable for use in breast pumps, where movement of the pump generating a cyclic vacuum suction force is suitable for manipulating (directly, or via another component manipulated by the pump) the polarization element. Further, polarization-based imaging is of particular interest, as this provides deeper optical penetration of the breast tissue, enabling potential imaging for pathologies not possible by standard methods. That is, certain pathologies of the breast occur at a depth in the tissue of the breast that typical imaging techniques cannot achieved (compared to polarization-based imaging). Therefore, a breast pump with a built-in polarization element having a polarization parameter that changes with operation of the breast pump may prove useful.

According to other examples in accordance with the invention there is provided a system for imaging tissue of the subject, comprising the personal care device according to an embodiment of the invention, a light source configured to emit light toward tissue of the subject via the polarization element, and a light or optical sensor configured to detect light transmitted and/or backscattered by the tissue, the detected light corresponding to the emitted light.

Of course, the personal care device may further include components required to perform imaging (rather than requiring the sensor and/or light source externally to the personal care device). Thus, this embodiment provides a means to image the breast tissue with high horizontal resolution by virtue of the polarization element having a polarization parameter that changes with movement of the movable component.

In some embodiments, the system may further comprise a controller configured to control the light source to emit light, and control the light sensor to detect transmitted and/or backscattered light corresponding to the emitted light. In this case, the system may further comprise a processor configured to process the detected light to generate an image of the tissue of the subject.

Preferably, the light source may be configured to emit light in any of infrared, near-infrared, and visible light spectrums.

According to examples in accordance with a further aspect of the invention there is provided a method for operating a personal care device comprising a movable component and an actuator. The method comprises moving, by the actuator, the moveable component; and changing, by the movement, a polarization parameter of a polarization element, the polarization element configured to polarize light incident on the polarization element.

According to additional examples in accordance with another aspect of the invention there is provided a computer program product comprising computer program code means which, when executed on a computing device having a processing system, cause the processing system to perform all of the steps of the method for operating a personal care device comprising a movable component and an actuator according to an embodiment of the invention.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents a simplified system block diagram of a personal care device according to an embodiment of the invention;
Fig. 2 presents a further simplified block diagram of a flow diagram of a system for imaging tissue of a subject according to another embodiment of the invention;
Fig. 3 presents a flow diagram of a method for operating a personal care device according to another aspect of the invention; and
Fig. 4 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Proposed concepts aim to provide schemes, solutions, concepts, designs, methods, and systems pertaining to a personal care device. Specifically, movement of a moveable component of the personal care device is utilized to change/alter a polarization of a polarization element. Thus, light on the polarization element may be polarized differently depending on movement of the moveable component. In some cases, the polarization element having a varying polarization parameter may be used for repeated, different polarization-based imaging of a tissue of a subject to which the personal care device is applied, potentially resulting in an image of the subject having a high horizontal resolution.

That is, movement of a movable component (moved by an actuator) of the personal care device is utilised in order to change a polarization parameter of a polarization element. This may be achieved by altering the dimensions, rotating, and/or filling a cavity of the polarization filter using the movement of the movable component. As such, a personal care device may be provided that has a polarization element attached thereto that differently polarizes incident light based on movement of the movable component.

One benefit arising from such an arrangement is the facilitation of multi-polarization imaging. In other words, the polarization filter may have a number of different polarization modes (corresponding to different polarization parameters). When an arrangement including a light source and light detector/sensor is provided, light incident to a surface via the polarization filter may be sensed to obtain an image. As the moveable component moves, the polarization parameter of the polarization element changes, and so the polarization of light incident to the surface may change. Thus, imaging with many different polarizations may be facilitated by embodiments of the invention.

By way of brief explanation, by using polarized light as opposed to non-polarized light, an increase in optical depth penetration may be achieved. That is, the light provided via the polarization filter may be used to penetrate a surface (e.g. tissue of a user of the personal care device) deeper than non-polarized light. For example, depth penetration of breast tissue may increase from 1-2 mm in typical optical methods, to 6-8mm when using polarized light. This is particularly beneficial for surfaces having objects/features of interest beneath the surface. Once again, this is useful for breast tissue, in which structures may develop deep within the tissue, and in which nipple ducts are often present up to 5mm deep.

However, polarization-based imaging usually has limited resolution in comparison to other optical methods. This problem may be effectively overcome by imaging the same surface with light of different polarizations. In other words, by illuminating a surface with light of various different polarizations, and detecting the various light after reflecting from the surface, an image with higher resolution may be acquired.

Accordingly, the invention facilitates this multi-polarization imaging by exploiting the movement of a movable component of the personal health device. That is, actuation of the personal care device (e.g. actuation of a breast pump) may be leveraged as the driving force to adapt either the degree of polarization of a polarization element, or the relative orientation of a polarization element. Furthermore, (additionally or alternatively) this modifiable polarization parameter may be utilised to suitably provide different polarized light to different surfaces, as desired. Hence, providing a personal care device having a polarization element with polarization parameter changeable with movement of a movable component may be advantageous for many applications.

The actuator and/or moveable component may be the actual mechanism that forms part of the primary function/operation of the personal care device (e.g., the rotation of a motor within an electric toothbrush, the piston of a pump of a breast pump, etc.). Thus, (periodic, cyclic, or oscillating) movement of the typical personal care device itself can be used to alter the polarization parameter of the polarization element. This may be particularly advantageous where imaging (via the polarization element) is performed synchronously with movement of the personal care device. That is, when the polarization parameters are to be dynamically adjusted in a synchronous manner with the personal care device, it may be beneficial to leverage the movement of the actuator and/or movable component that is responsible for operation of the personal care device.

By way of illustrative example, during pumping of a breast pump, skin tissue is subject to cyclic shear and tensile deformation (i.e., inducing a change in collagen fibre orientation within the tissue). As a result, the pumping action alters the polarization state of incident light interacting with the deformed tissue. A similar concept is leveraged by the present invention, except that the pumping action (more generally, the movement of the moveable component by the actuator) is used to change polarization parameters of a polarization element (rather than the accidental consequence of changing polarization characteristics of skin tissue). For example, the orientation of fixed polarizers and/or the degree of polarization of the polarizing component is changed.

Thus, it is proposed to use the movement of the moveable component (e.g. pumping action, full rotary action, low-amplitude oscillation, etc.) to actuate a change in polarization parameters. The movement could be adapted to, for example:
(i) Increase a thickness of the polarizing element by compressing sides of the polarizing element. As a result, the degree of polarization is changed as the total polarization is determined by thickness x refractive index asymmetry;
(ii) Providing fluid (liquid, gas or liquid-gas mixture) into a cavity of the polarizing element to alter the optical path (provided the liquid has a different refractive index to the material previously inside the cavity);
(iii) Increasing a physical dimension of the polarizing element (e.g., stretch the polarizer), altering the degree of polarization similarly to (i); and/or
(iv) Rotate the polarizing element to rotate clockwise/anticlockwise direction.

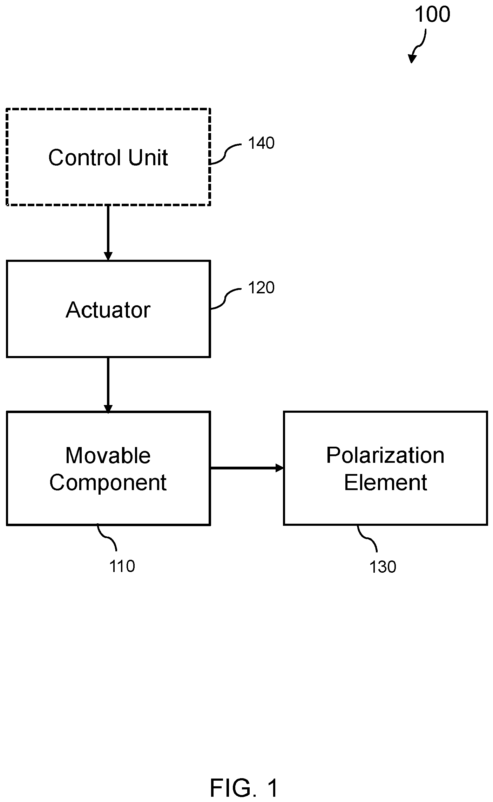

Fig. 1 presents a simplified system block diagram of a personal care device according to an embodiment of the invention. The personal care device comprises a movable component 110, an actuator 120, a polarization element 130, and an optional control unit 140. Of course, the personal care device 100 may comprise further features/components, required to perform the primary personal care function. For example, the personal care device 100 may be a breast pump, an oral care device such as a toothbrush, a mouthpiece, a shaver, a tissue massager, a hairdryer, a grooming device etc. Therefore, the personal care device 100 may comprise further components associated with such devices.

More specifically, the actuator 120 is configured to move the movable component 110. That is the personal care device 100 comprises an actuator 120/element that causes the personal care device is operate, which additionally/alternatively causes the movable component 110 to move. Said movement of the movable component 110 changes a polarization parameter of the polarization element 130. The means by which the polarization parameter of the polarization element 130 may be changed will be described in more detail below.

Importantly, the polarization element 130 that is configured to polarize light incident on the polarization element 130. The polarization element/device 130 may be a polarization filter, a liquid crystal element, or any other element that is suitable for polarizing incident light. The polarization element 130 will have various polarization parameters/characteristics. This may include, for example, a degree of polarization and/or a type of polarization (e.g. linear, right circular, left circular).

In other words, the movement of the movable component 110 caused by the actuator 120 is used to manipulate/adjust the polarization element 130, such that a polarization parameter of the polarization filter is changed by said movement.

The actuator 120 may be any device that causes the personal care device to operate. Accordingly, the actuator 120 may be/may include a motor and a shaft, or may be a component that changes shape under an applied voltage (e.g. a thin film actuator such as an electroactive polymer). Essentially, any component of the personal care device that is responsible for at least a part of the movement or control of a mechanism of the personal care device may be the actuator 120.

Of course, in some embodiments the movable component 110 may be incorporated in, or be the polarizing element itself. Put another way, the actuator 120 (or a moveable component thereof) may connect directly to the polarization element 130 to change the polarization parameter of polarization element 130.

The polarization element 130 and movable component 110 may be configured in a number of ways so that movement of the movable component 110 changes a polarization parameter.

Firstly, the polarization element 130 may be flexible/elastic such that one or more dimensions of the polarization element 130 may be changed. That is, the polarization element 130 may be compressed, stretched or otherwise altered as a result of movement of the moveable component so that one or more dimensions of the polarization element 130 are altered. As a result, the polarization element 130 may be made thinner, slimmer, shorter, thicker, wider or taller. Simply put, a shape of the polarization element 130 may be changed.

Accordingly, a degree of polarization of light incident to the polarization element 130 may be altered. Thus, the polarization parameter relating to an extent/degree/magnitude of polarization may be changed. In some cases, this may be due to a change in the length of an optical path of light incident to the polarization element 130.

Alternatively, or in addition to configuring the polarization so as to be flexible, the actuator 120 may be configured to move the moveable component so as to rotate the polarization element 130. By changing the orientation of the polarization element 130, an angle of polarization may be changed. Instead, a type of polarization may be changed by exposing at least a part of a face of the polarization element 130 to incident light that differently polarizes light. Simply put, by rotating the polarization element 130 may alter the polarization parameter in a number of different ways.

Furthermore, the polarization parameter may be changed by altering an aspect of the polarization element 130 to change a length of the optical path for light incident to the polarization element 130.

In one case, the polarization element 130 may comprise a cavity/void therein for containing a fluid (i.e. a liquid, gas, or liquid-gas mixture). Thus, a length of an optical path of the polarization element 130 may depend on a volume of liquid within the polarization element 130. Introducing the liquid into the cavity will change the optical characteristics of the polarization element 130, and thus a polarization parameter.

Specifically, the liquid may have polarization properties, e.g. a liquid crystal. Thus, introduction of the liquid may change a polarization parameter of the polarization element 130. Additionally, the cavity may optionally comprise one or more surfaces adapted to align the liquid (as is the case with liquid crystal in a typical LCD). This may help to improve the predictability, and reliability of the change in the polarization parameter with movement of the movable component 110.

Thus, additionally or alternatively to the change in dimension and orientation of the polarization as described above, the actuator 120 may be configured to move the movable component 110 so as to change the volume of liquid in the cavity.

The personal care device may also comprise a control unit 140. The control unit 140 may be configured to manipulate the polarization element 130 to change the polarization parameter by controlling the actuator 120 to move the movable component 110. In other words, the control unit 140 may achieve a target/desired change in the polarization parameter by appropriately controlling/operating the actuator 120 to move the movable component 110. Without the control unit 140, the polarization parameter may passively change as the actuator 120 moves the movable component 110, whereas with the control unit 140, movement of the movable component 110 by the actuator 120 may be deliberately selected so as to give rise to a desired polarization parameter change.

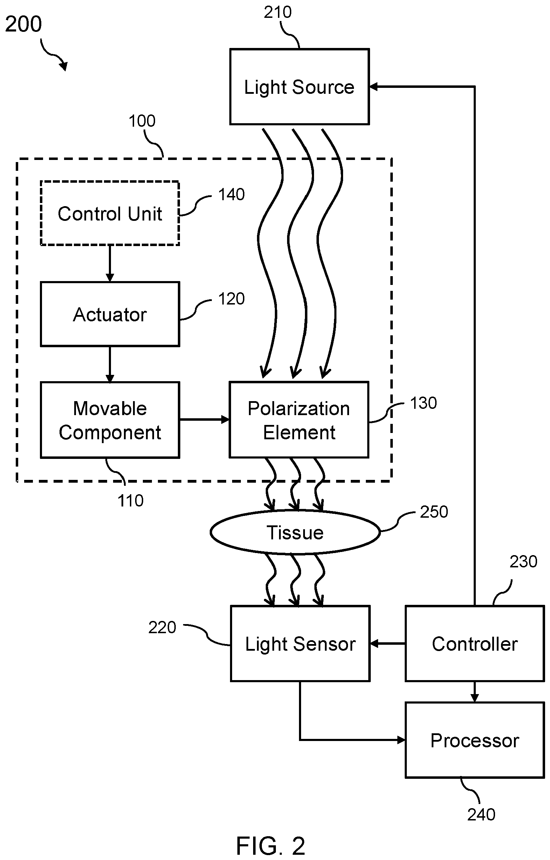

Fig. 2 presents a further simplified block diagram of a flow diagram of a system for imaging tissue of a subject according to another embodiment of the invention. This system presents one use of the personal care device depicted in Fig. 1. In particular, the system comprises a light source 210, and a light sensor 220 in addition to the personal care device 100 of Fig. 1.

To be clear, the system 200 may also be incorporated in a personal care device 100 alongside the movable component 110, actuator 120, and polarization element 130. However, the system 200 may be partially or wholly separate from the personal care device 100. For example, the personal care device may also comprise the light source 210 and light sensor 220, but may not include the controller 230 and processor 240 (which may be provided on a separate device and connected to the light source and sensor wirelessly).

The depicted system 200 can be used for polarization-based imaging of an area corresponding to a use site of the personal care device. Specifically, the system may be used to image tissue 250 of the subject using polarized light. Therefore, the system may be able to image tissue 250 at larger depths than by standard imaging methods. Further, due to the changeability of the polarization parameter by the moveable component/actuator 120, a lateral resolution of the resultant image may be improved.

By way of explanation, the light source 210 is configured to emit light toward tissue 250 of the subject via the polarization element 130. In other words, the light source 210 emits light toward the polarization element 130, which is polarized and is subsequently transmitted and/or backscattered by tissue 250 of the subject.

The light source 210 may be configured to emit monochromatic light toward the tissue 250 of the subject. That is, the light source 210 may be configured to irradiate tissue 250 of the subject with monochromatic light. Monochromatic light in this context means light of a single wavelength, or a narrow band of wavelengths.

For example, the light source 210 may comprise a broadband light emitter (e.g. an LED) with a light filter allowing only monochromatic light from the broadband light emitter. The light filter, in some cases, may be operable in multiple modes to let monochromatic light of different wavelengths through. Alternatively, the light source 210 may simply comprise a light emitter configured to emit monochromatic light. The light source 210 may be LED based, or may be some other light source 210 suitable for providing light for polarization.

More particularly, the light source 210 may optionally comprise a light emitter configured to emit light in the infrared, near-infrared, and/or visible light spectrums, and a variable light frequency filter configured to receive the emitted light, and output monochromatic light toward the tissue 250 of the subject.

A light sensor 220 is provided, and configured to detect the light transmitted and/or backscattered by the tissue 250. To avoid any confusion, the detected light corresponds to the emitted light from the tissue 250.

In some cases, it may be beneficial for a further polarization element (not depicted) to be provided to receive light after being backscattered/transmitted by the tissue, and to output light from this polarization element to the light sensor. Said polarization element may have a polarization parameter modifiable/alterable by the moveable component (similar to polarization element 130), may be alterable by other means, or may be fixed.

The light sensor 220, for example, may be a CMOS sensor or other camera system that can detect light in at least one of the visible, infrared, and near infrared spectrums. Essentially, the light sensor 220 may be any component that can convert incident light into a signal describing the incident light for further analysis.

The light sensor 220 may be appropriately positioned so as to detect the backscattered and/or transmitted light. In some cases, it may be preferable that the light sensor 220 is positioned to only detect backscattered light, which lends itself to a more compact system (i.e. does not require the sensor and source to be positioned opposite sides of the tissue 250).

Also, the light sensor 220 and/or source may comprise suitable optics for gathering/guiding light toward a component for sensing the light. Of course, the system may further comprise one or more lens elements (not depicted) to facilitate the appropriate manipulation of the light beam toward the polarization element 130 and tissue 250, and to gather a refocus the light transmitted/backscattered by the tissue 250 onto the light sensor 220.

In this way, the light sensor 220 may gather information corresponding to the emitted light. Thus, an image of the tissue 250 may be acquired by the light sensor 220. The gathered information may relate to light polarized in one or more different ways by the polarization element 130 as the movable component 110 moves.

In some embodiments, the system may additionally comprise a controller 230 configured to control the light source 210 to emit light, and control the light sensor 220 to detect transmitted and/or backscattered light corresponding to the emitted light. They system may then also comprise a processor 240 configured to process the detected light to generate an image of the tissue 250 of the subject.

In other words, the controller 230 is configured for controlling the operation of the above-mentioned components. Specifically, at its simplest, the controller 230 is configured to control the light source 210 to emit light, and control the light sensor 220 to detect light corresponding to the emitted light. That is, the controller 230 prompts the light source 210 to emit light toward the tissue 250 (via the polarization element 130), and prompts the light sensor 220 to detect the emitted light (after being transmitted through the polarization element 130, and backscattered and/or transmitted by the tissue 250.

Subsequently, the processor 240 is configured to process the detected light to generate an image of the tissue 250 of the subject. In other words, the processor 240 processes/analyses a signal describing the light detected by the light sensor 220 in order to generate an image of (at least part) of the tissue 250 of the subject.

Of course, the controller 230 may prompt the light source 210 and light sensor 220 to perform the above many times. This may be performed for various parts of the tissue 250 of the subject, and the resultant light signal used to produce a complete (or more complete) image of the tissue 250 of the subject. Further, this may be performed for various different polarizations as the polarization parameter of the polarization element 130 changes with movement.

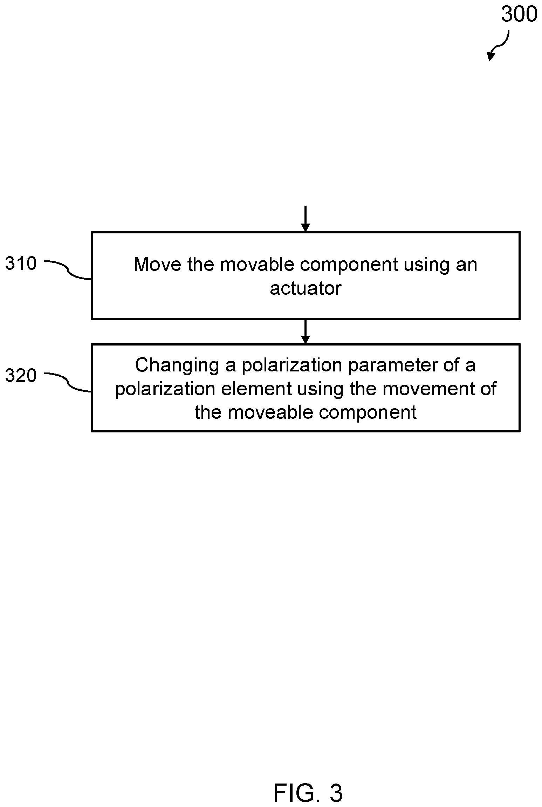

Moving on, Fig. 3 presents a flow diagram of a method 300 for operating the personal care device of Fig. 1 according to another aspect of the invention.

Specifically, the personal care device comprises a movable component 110 and an actuator 120 configured to move the movable component 110. The personal care device must also comprise a polarization element 130 having a polarization parameter modifiable by movement of the movable component 110.

In step 310, the moveable component is moved by the actuator. This movement may occur during usually/typical operation of the device (e.g. during pumping action of a breast pump), or may be separately controlled. The movement may be a linear motion, which may be suitable for altering dimension of the polarization element, or may be a rotary/circular motion, which may be suitable for altering an orientation of the polarization element. Of course, methods of changing one type of movement into another suitable type of movement would be readily appreciated by the skilled person.

In step 320, a polarization parameter of the polarization element is changed by the movement. In this way, the polarization element is configured to polarize light incident on the polarization element differently as the movable component moves. For example, a degree and/or type of polarization may change as the movable component moves.

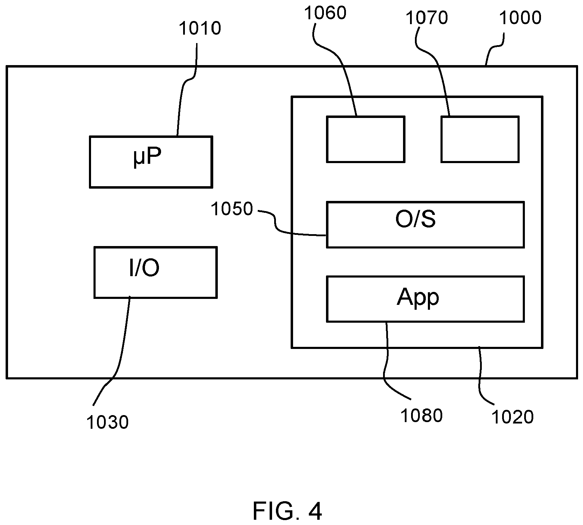

Fig. 4 illustrates an example of a computer 1000 within which one or more parts of an embodiment may be employed. Various operations discussed above may utilize the capabilities of the computer 1000. For example, one or more parts of a system for imaging tissue of a subject may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, smart phones, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, functional programming and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs. 1 and 3, and the system described in relation to Fig. 2, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out a method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram Figs. 1 and 2 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A personal care device (100), comprising:
a movable component (110);
an actuator (120) configured to move the movable component;
a polarization element (130) configured to polarize light incident on the polarization element; and
wherein the movement of the movable component changes a polarization parameter of the polarization element.

2. The personal care device of claim 1, wherein the polarization element (130) is flexible such that one or more dimensions of the polarization element may be changed, thereby changing a degree of polarization of light incident to the polarization element.

3. The personal care device of claim 2, wherein the actuator (120) is configured to move the movable component (110) so as to compress the polarization element (130).

4. The personal care device of claim 2 or 3, wherein the actuator (120) is configured to move the moveable component (110) so as to stretch the polarization element (130).

5. The personal care device of any of claims 1-4, wherein the actuator (120) is configured to move the moveable component (110) so as to rotate the polarization element (130).

6. The personal care device of any of claims 1-5, wherein the polarization element (130) comprises a cavity for containing a fluid, a length of an optical path of the polarization element depending on a volume of fluid within the polarization element, and
wherein the actuator (120) is configured to move the movable component (110) so as to change the volume of fluid in the cavity.

7. The personal care device of any of claims 1-6, wherein the actuator (120) and/or the movable component (110) is further configured for operation of the personal care device.

8. The personal care device of any of claims 1-7, further comprising a control unit (140) configured to manipulate the polarization element (130) to change the polarization parameter by controlling the actuator (120) to move the movable component (110).

9. The personal care device of any of claims 1-8, wherein the actuator (120) and/or movable component (110) is a pump.

10. The personal care device of any of claims 1-9, wherein the personal care device is a breast pump, and wherein the actuator (120) and/or movable component (110) is a pump configured to move the moveable component.

11. A system (200) for imaging tissue of a subject, comprising:
the personal care device (100) of any of claims 1-10;
a light source (210) configured to emit light toward tissue of the subject via the polarization element (130); and
a light sensor (220) configured to detect light transmitted and/or backscattered by the tissue, the detected light corresponding to the emitted light.

12. The system of claim 11, further comprising a controller (230) configured to:
control the light source to emit light; and
control the light sensor to detect transmitted and/or backscattered light corresponding to the emitted light; and
a processor (240) configured to process the detected light to generate an image of the tissue of the subject.

13. The system of claim 11 or 12, wherein the light source (210) is configured to emit light in any of infrared, near-infrared, and visible light spectrums.

14. A method (300) for operating a personal care device comprising a movable component and an actuator, comprising:
moving (310), by the actuator, the moveable component; and
changing (320), by the movement, a polarization parameter of a polarization element, the polarization element configured to polarize light incident on the polarization element.

15. A computer program product comprising computer program code means which, when executed on a computing device having a processing system, cause the processing system to perform all of the steps of the method according claim 14.
